(19) Europäisches Patentamt
European Patent Office
Office européen des brevets

(11) Numéro de publication: **0 171 342**
**B1**

(12) # FASCICULE DE BREVET EUROPEEN

(45) Date de publication du fascicule du brevet: **07.10.87**

(51) Int. Cl.[4]: **C 07 C 97/07,** C 07 C 143/78, A 61 K 31/135

(21) Numéro de dépôt: **85450014.7**

(22) Date de dépôt: **19.06.85**

(54) **Nouvelles (amino-1 alkylidényl)-2 indanediones-1,3 substituées, leur méthode de préparation et leur application thérapeutique.**

(30) Priorité: **20.07.84 FR 8411679**
**23.05.85 FR 8507922**

(43) Date de publication de la demande:
**12.02.86 Bulletin 86/7**

(45) Mention de la délivrance du brevet:
**07.10.87 Bulletin 87/41**

(84) Etats contractants désignés:
**BE CH DE GB IT LI LU NL**

(56) Documents cités:
**CHEMICAL ABSTRACTS, vol. 96, no. 11, 15 mars 1982, page 555, réf. no. 85189y, et Chemical Substance Index, janvier-juin 1982, page 3410CS, Columbus, Ohio, US; MATSUO, TOSHIYASU et al.: "Studies on tenuazonic acid analogs. V. Syntheses and antitumor activities of indan-1,3-dione derivatives"**

(73) Titulaire: **SOCIETE CORTIAL S.A., 7 rue de l'Armorique, F-75015 Paris (FR)**

(72) Inventeur: **Le Baut, Guillaume, Prof., 5 rue de la Baugerie, F-44230 St-Sébastien-sur-Loire (FR)**
Inventeur: **Sparfel, Louis, Prof., 8 rue François Marchais, F-44400 Reze-lès-Nantes (FR)**
Inventeur: **Creuzet, Marie-Hélène, Jardin de Gambetta T3, F-33000 Bordeaux (FR)**
Inventeur: **Feniou, Claude, 5 rue Général Guillaumat, F-33600 Pessac (FR)**
Inventeur: **Pontagnier, Henri, 21 rue Edouard-Vaillant, F-33600 Pessac (FR)**
Inventeur: **Prat, Gisèle, Hameau de Noailles Villa 18, F-33400 Talence (FR)**

(74) Mandataire: **Tajan, Marie-Thérèse, LABORATOIRES SARGET Avenue du Président JF Kennedy, F-33701 Mérignac (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne des (amino-1 alkylidényl)-2 indanediones-1,3 substituées, leur méthode de préparation et leur application thérapeutique.

Les produits faisant l'objet de la présente invention ont pour formule générale

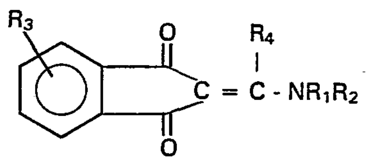

(I)

dans laquelle

$R_1$ représente un atome d'hydrogène ou un groupement alkyl inférieur tel que méthyl,

$R_2$ représente un atome d'hydrogène ou un groupement alkyl inférieur de $C_1$ à $C_4$ linéaire ou ramifié choisi dans le groupe formé par méthyl, éthyl, propyl, isopropyl, butyl, isobutyl, tertiobutyl, ou bien un groupement alcoxyéthyl, de préférence méthoxyéthyl, ou bien un groupement cycloalkyl choisi dans le groupe formé par cyclopropyl, cyclobutyl, cyclopentyl, cyclohexyl et cycloheptyl, ou bien un groupement cycloalkylméthyl, de préférence cyclopropylméthyl, ou bien un groupement benzyl substitué ou non sur le cycle aromatique par un ou plusieurs substituants choisis parmi chloro, bromo, fluoro, alkyl inférieur de $C_1$ à $C_4$, alcoxy inférieur de $C_1$ à $C_4$, trifluorométhyl,

$R_3$ représente un atome d'hydrogène ou un ou plusieurs substituants, identiques ou différents, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl.

Les produits de formule générale (I) sont des produits nouveaux. On connaît des produits analogues tels que ceux décrits dans les travaux de Gasjuna L. et coll. Latv. P. S. R. Zinat. Akad. Vest. Kim. Ser. 1980, 1, 98-101. Aucune application thérapeutique n'a toutefois été décrite pour ces dérivés.

Nous avons maintenant découvert que les produits selon la formule (I) sont utiles en thérapeutique humaine et vétérinaire en tant que diurétiques dans le traitement des oedèmes et de l'hypertension artérielle et présentent également d'autres propriétés notamment antiinflammatoires, bronchodilatatrices, anticholinergiques, antispasmodiques, anticalciques, antidépressives, antiépileptiques, antihypertensives, anticalciques, anti$NH_2$.

Les produits de formule générale (I) sont préparés des façon générale, dans le cas où $NR_1R_2$ est différent de $NH_2$, par une réaction mettant en jeu une indanedione-1,3 de formule générale

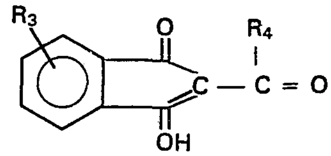

(II)

dans laquelle

$R_3$ représente un atome d'hydrogène ou un ou plusieurs substituants, identiques ou différents, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl,

et l'amine de formule générale $HNR_1R_2$ dans laquelle $R_1$ représente un atome d'hydrogène ou un groupement alkyl inférieur tel que méthyl, et $R_2$ représente un groupement alkyl inférieur de $C_1$ à $C_4$ linéaire ou ramifié choisi dans le groupe formé par méthyl, éthyl, propyl, isopropyl, butyl, isobutyl, tertiobutyl, ou bien un groupement alcoxyéthyl, de préférence méthoxyéthyl, ou bien un groupement cycloalkyl choisi dans le groupe formé par cyclopropyl, cyclobutyl, cyclopentyl, cyclohexyl et cycloheptyl, ou bien un groupement cycloalkylméthyl, de préférence cyclopropylméthyl, ou bien un groupement benzyl substitué ou non sur le cycle aromatique par un ou plusieurs substituants choisis parmi chloro, bromo, fluoro, alkyl inférieur de $C_1$ à $C_4$, alcoxy inférieur de $C_1$ à $C_4$, trifluorométhyl, en présence d'acide formique en solution dans un solvant tel que l'éthanol. Une variante consiste à faire réagir le produit de formule (II) et le chlorhydrate de l'amine $HNR_1R_2$ définie plus haut, en présence d'un accepteur d'hydracide tel que le carbonate de sodium.

Les produits de formule générale (I) tels que $NR_1R_2 = NH_2$ sont préparés de façon générale par chauffage à une température comprise entre 80 et 100°C du mélange constitué par un solvant, de préférence l'éthanol, l'ammoniac, et une indanedione-1,3 de formule générale (II) dans laquelle $R_3$ représente un atome d'hydrogène ou un ou plusieurs substituants, identiques ou différents, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl.

Les exemples exposés ci-après vont permettre de mieux comprendre la présente invention sans toutefois en limiter la portée.

*EXEMPLE I*

(Méthylamino-1 éthylidène)-2 indanedione-1,3; produit de formule (I) dans laquelle $R_1 = R_3 = H$ et $R_2 = R_4 = CH_3$ ; nom de code COR3760.

*Préparation*

A une solution de 0,2 ml d'acide formique, 5 ml de

solution éthanolique de méthylamine à 25% et 20 ml d'éthanol, chauffée vers 90°C, on ajoute petit à petit en 4 heures une solution de 1 g d'acétyl-2 indanedione-1,3 dans 40 ml d'éthanol. Les 2/3 du solvant sont éliminés par évaporation. Après refroidissement, le précipité est filtré, lavé à l'éthanol froid et recristallisé dans l'éthanol. Le rendement est de 76,4%.

*Propriétés physicochimiques*

Point de fusion: 200°C (éthanol).

Spectre de RMN dans $CDCl_3$: 10,77 ppm, singulet, 1 proton, NH + OH; 7,62 ppm, multiplet, 4 protons, H aromatiques phtaloyle; 3,15 et 3,08 ppm, 2 singulets, 3 protons, $-N-CH_3$; 2,65 ppm, singulet, 3 protons, $CH_3$.

*EXEMPLE II*

Chloro-5 (méthylamino-1 éthylidène)-2 indanedione-1,3; produit de formule (I) dans laquelle $R_1$ = H, $R_2$ = $R_4$ = $CH_3$ et $R_3$ = Cl en 5; nom de code COR3773.

*Préparation*

A une solution de 0,2 ml d'acide formique, 5 ml de solution éthanolique de méthylamine à 25% et 20 ml d'éthanol, chauffée vers 90°C, on ajoute petit à petit en 4 heures une solution de 1 g d'acétyl-2 chloro-5 indanedione-1,3 dans 45 ml d'éthanol. Le mélange réactionnel est maintenu à chaud pendant 4 heures. Le solvant est éliminé par évaporation. Le résidu est purifié par passage sur colonne de gel de silice en éluant par du chlorure de méthylène. Le rendement est de 66%.

*Propriétés physicochimiques*

Point de fusion: 192°C (chlorure de méthylene).

Spectre de RMN dans $CDCl_3$: 10,83 ppm, singulet, 1 proton, NH + OH; 7,70 ppm, multiplet, 3 protons, H aromatiques phtaloyle; 3,20 et 3,10 ppm, 2 singulets, 3 protons, $-N-CH_3$; 2,67 ppm, singulet, 3 protons, $CCH_3$.

*EXEMPLE III*

Chloro-5 (éthylamino-1 éthylidène)-2 indanedione-1,3; produit de formule (I) dans laquelle $R_1$ = H, $R_2$ = $C_2H_5$, $R_4$ = $CH_3$ et $R_3$ = Cl en 5; nom de code COR3774.

*Préparation*

A une solution de 0,2 ml d'acide formique, 5 ml de solution aqueuse d'éthylamine à 70% et 20 ml d'éthanol, chauffée vers 90°C, on ajoute petit à petit en 4 heures une solution de 1 g d'acétyl-2 chloro-5 indanedione-1,3 dans 50 ml d'éthanol. Le mélange réactionnel est maintenu à chaud pendant 4 heures. Le solvant est éliminé par évaporation. Le résidu est purifié par passage sur colonne de gel de silice en éluant par du chlorure de méthylène. Le rendement est de 57%.

*Propriétés physicochimiques*

Point de fusion: 142°C (chlorure de méthylene).

Spectre de RMN dans $CDCl_3$: 10,87 ppm, singulet, 1 proton, NH; 7,62 ppm, multiplet, 3 protons, H aromatiques phtaloyle; 3,52 ppm, multiplet, 2 protons, $CH_2$; 2,65 ppm, singulet, 3 protons, $CH_3$; 1,38 ppm, singulet, 3 protons, $CH_3$.

*EXEMPLE IV*

(Diméthylamino-1 éthylidène)-2 indanedione-1,3; produit de formule (I) dans laquelle $R_1$ = $R_2$ = $R_4$ = $CH_3$ et $R_3$ = H; nom de code COR3788.

*Préparation*

A une solution de 0,3 ml d'acide formique, 5 ml de solution éthanolique de méthylamine à 25% et 20 ml d'éthanol, chauffée vers 80°C, on ajoute très lentement une solution éthanolique de 3 g d'acétyl-2 indanedione-1,3. Le chauffage est poursuivi pendant deux jours. Le solvant est éliminé par évaporation. Le résidu est recristallisé dans l'éthanol. Le rendement est de 75%.

*Propriétés physicochimiques*

Point de fusion: 164°C (éthanol).

Spectre IR: bandes ν CO à 1685 et 1640 $cm^{-1}$.

Spectre de RMN dans DMSO $D_6$: 7,60 ppm, multiplet, 4 protons, H aromatiques phtaloyle; 2,75 ppm, singulet, 6 protons, $N-CH_3$; 2,38 ppm, singulet, 3 protons, $CCH_3$.

*EXEMPLE V*

(Ethylamino-1 éthylidène)-2 indanedione-1,3; produit de formule (I) dans laquelle $R_1$ = $R_3$ = H, $R_4$ = $CH_3$ et $R_2$ = $C_2H_5$; nom de code COR3789.

*Préparation*

A une solution de 0,25 ml d'acide formique, 2 ml de solution aqueuse d'éthylamine à 70% et 20 ml d'éthanol, chauffée vers 80-90°C, on ajoute très lentement une solution de 2 g d'acétyl-2 indanedione-1,3 dans 100 ml d'éthanol. Le chauffage est poursuivi au reflux de l'éthanol pendant 2 jours. Le solvant est éliminé par évaporation. Le résidu est purifié par passage sur colonne de gel de silice en éluant par du chlorure de méthylène. Le produit est recristallisé dans l'éthanol. Le rendement est de 84%.

*Propriétés physicochimiques*

Point de fusion: 138°C (éthanol).

Spectre IR: bandes ν C = O à 1685 et 1645 $cm^{-1}$.

Spectre de RMN dans $CDCl_3$: 7,66 ppm, multiplet, 4 protons, H aromatiques phtaloyle; 3,47 ppm, multiplet, 2 protons, $CH_2$; 2,67 ppm, singulet, 3 protons, $CH_3$; 1,35 ppm, singulet, 3 protons, $CH_3$.

*EXEMPLE VI*

(Isopropylamino-1 éthylidène)-2 indanedione-1,3; produit de formule (I) dans laquelle $R_1$ = $R_3$ = H, $R_4$ = $CH_3$ et $R_2$ = $CH(CH_3)_2$; nom de code COR3792.

*Préparation*

A une solution de 0,25 ml d'acide formique, 2 ml d'isopropylamine et 20 ml d'éthanol, chauffée vers 80°C, on ajoute très lentement une solution éthanolique de 2 g d'acétyl-2 indanedione-1,3. Le chauffage est poursuivi pendant 2 jours. Le solvant est éliminé par évaporation. Le résidu est purifié par passage sur colonne de gel de silice en éluant par du

chlorure de méthylène. Le produit ainsi obtenu est recristallisé dans 15 ml d'éther isopropylique. Le rendement est de 74%.

*Propriétés physicochimiques*

Point de fusion: 88°C (éther isopropylique).
Spectre IR: bandes ν (C=O) à 1685 et 1635 $cm^{-1}$.

Spectre de RMN dans $CDCl_3$: 10,75 ppm, singulet, 1 proton, NH; 7,38 ppm, multiplet, 4 protons, H aromatiques phtaloyle; 3,95 ppm, septuplet, CH; 2,67 ppm, singulet, 3 protons, $CH_3$; 1,35 ppm, doublet, 6 protons, $CH_3$.

*EXEMPLE VII*

(Tertiobutylamino-1 éthylidène)-2 indanedione-1,3; produit de formule (I) dans laquelle $R_1 = R_3 = H$, $R_2 = C(CH_3)_3$, $R_4 = CH_3$; nom de code COR4001.

*Préparation*

A une solution de 0,25 ml d'acide formique, 25 ml d'éthanol et 5 ml de tertiobutylamine, chauffée à 80-90°C, on ajoute très lentement une solution de 1,5 g d'acétyl-2 indanedione-1,3 dans 150 ml d'éthanol. Le chauffage est poursuivi pendant 2 jours. Le solvant est éliminé par évaporation. Après refroidissement, le précipité est filtré et recristallisé dans un mélange éther isopropylique-éthanol(5/2). Le rendement est de 77%.

*Propriétés physicochimiques*

Point de fusion: 187°C [mélange éther isopropylique-éthanol(5/2)]. Le produit se sublime vers 175°C.
Spectre IR: bandes ν C = O à 1705 et 1685 $cm^{-1}$; bandes ν $NH^+$ à 3030, 2840, 2650, 2450 $cm^{-1}$.
Spectre de RMN dans $CDCl_3$: 7,6 - 7,3 ppm, multiplet, 4 protons, H aromatiques phtaloyle; 5,45 ppm, multiplet, 1 proton, NH; 2,38 ppm, singulet, 3 protons, $CH_3$ éthylidénylique; 1,08 ppm, singulet, 9 protons, $(CH_3)_3$.

*EXEMPLE VIII*

Nitro-5 (méthylamino-1 éthylidène)-2 indanedione-1,3; produit de formule (I) dans laquelle $R_1 = H$, $R_2 = CH_3$, $R_3 = NO_2$ en 5 et $R_4 = CH_3$; nom de code COR4002.

*Préparation*

A une solution de 0,25 ml d'acide formique, 5 ml de solution éthanolique de méthylamine à 25% et 25 ml d'éthanol, chauffée vers 80-90°C, on ajoute goutte à goutte une solution de 1,75 g d'acétyl-2 nitro-5 indanedione-1,3 dans 200 ml d'éthanol. Le chauffage est poursuivi pendant 2 jours. Le solvant est éliminé par évaporation. Le résidu est purifié par passage sur colonne de gel de silice en éluant par du chlorure de méthylène. Le produit est recristallisé dans l'éthanol. Le rendement est de 63%.

*Propriétés physicochimiques*

Point de fusion: 212°C (éthanol).
Spectre IR: bandes ν C = O à 1695 et 1650 $cm^{-1}$; bandes ν $NO_2$ à 1535 et 1350 $cm^{-1}$.
Spectre de RMN dans $CDCl_3$: 10,97 ppm, singulet, 1 proton, NH; 8,55-7,80 ppm, multiplet, 3 protons, H aromatiques phtaloyle; 3,22 ppm, 2 singulets, 3 protons, $-N-CH_3$; 2,70 ppm, singulet, 3 protons, $CCH_3$.

*EXEMPLE IX*

(Méthylamino-1 propylidényl)-2 indanedione-1,3; produit de formule (I) dans laquelle $R_1 = H$, $R_2 = CH_3$, $R_3 = H$ et $R_4 = C_2H_5$; nom de code COR4003.

*Préparation*

A une solution de 0,25 ml d'acide formique, 5 ml de solution éthanolique de méthylamine à 25% et 25 ml d'éthanol, chauffée à 90°C, on ajoute très lentement une solution de 2 g de propionyl-2 indanedione-1,3 dans 50 ml d'éthanol. Le chauffage du mélange réactionnel est poursuivi pendant 2 jours. Le solvant est éliminé par évaporation. Le produit obtenu est recristallisé dans l'éthanol. Le rendement est de 96%.

*Propriétés physicochimiques*

Point de fusion: 139,5°C (éthanol).
Spectre IR: bandes ν C = O à 1695, 1680, 1645 $cm^{-1}$; bande ν NH à 3230 $cm^{-1}$.
Spectre de RMN dans $CDCl_3$: 10,08 ppm, multiplet, 1 proton, NH; 7,67 ppm, multiplet, 4 protons, H aromatiques phtaloyle; 3,17 ppm, 2 singulets, 3 protons, $NCH_3$; 3,13 ppm, quadruplet, 2 protons, $CH_2$; 1,27 ppm, triplet, 3 protons, $CH_3$.

*EXEMPLE X*

(Alpha-méthylamino benzylidényl)-2 indanedione-1,3; produit de formule (I) dans laquelle $R_1 = H$, $R_2 = CH_3$, $R_3 = H$ et $R_4 = C_6H_5$; nom de code COR4004.

*Préparation*

A une solution de 0,25 ml d'acide formique, 5 ml de solution éthanolique de méthylamine à 25% et 20 ml d'éthanol, chauffée vers 90°C, on ajoute goutte à goutte une solution de 1,4 g de benzoyl-2 indanedione-1,3. Le chauffage est poursuivi pendant 2 jours. Le solvant est éliminé par évaporation. Le résidu est recristallisé dans l'éthanol. Le rendement est de 78%.

*Propriétés physicochimiques*

Point de fusion: 179°C (éthanol).
Spectre IR: bandes ν CO à 1695, 1670 et 1650 $cm^{-1}$; bande ν NH à 3200 $cm^{-1}$.
Spectre de RMN dans $CDCl_3$: 10,95 ppm, multiplet, 1 proton, NH; 7,95-7,28 ppm, multiplet, 9 protons, H aromatiques; 3 et 2,9 ppm, 2 singulets, 3 protons, $N-CH_3$.

*EXEMPLE XI*

Dichloro-5,6 (méthylamino-1 éthylidényl)-2 indanedione-1,3; produit de formule (I) dans laquelle $R_1 = H$, $R_2 = CH_3$, $R_3 = 2$ chlore en 5,6 et $R_4 = CH_3$; nom de code COR4006.

*Préparation*

A une solution de 0,4 ml d'acide formique, 10 ml de solution éthanolique de méthylamine à 25% et

30 ml d'éthanol, chauffée vers 80-90°C, on ajoute très lentement une solution de 2 g d'acétyl-2 dichloro-5,6 indanedione-1,3 dans l'éthanol. Le chauffage est poursuivi pendant 2 jours. Après refroidissement, on filtre. On recueille ainsi 1,96 g de produit jaune verdâtre. Ce résidu est purifié par passage sur colonne de gel de silice en éluant par du chlorure de méthylène. Le rendement est de 85%.

*Propriétés physicochimiques*

Point de fusion: 286°C (chlorure de méthylène).
Spectre IR: bandes ν C = O à 1690 et 1645 $cm^{-1}$.
Spectre de RMN dans DMSO $D_6$ à 250 MHz: 7,73 ppm, singulet, 2 protons, H aromatiques phtaloyle; 3,12 ppm, doublet, 3 protons, $NCH_3$; 2,58 ppm, singulet, 3 protons, $CH_3$.

*EXEMPLE XII*

Dichloro-5,6 (éthylamino-1 éthylidényl)-2 indanedione-1,3; produit de formule (I) dans laquelle $R_1$ = H, $R_2$ = $C_2H_5$, $R_3$ = 2 chlore en 5,6 et $R_4$ = $CH_3$; nom de code COR4007.

*Préparation*

A une solution de 0,2 ml d'acide formique, 5 ml de solution éthanolique d'éthylamine à 25% et 20 ml d'éthanol, chauffée vers 80-90°C, on ajoute très lentement une suspension éthanolique de 1,1 g d'acétyl-2 dichloro-5,6 indanedione-1,3. Le chauffage est poursuivi pendant 2 jours. Le solvant est éliminé par évaporation. Le résidu est purifié par passage sur colonne de gel de silice en éluant par du chlorure de méthylène. Le rendement est de 95%.

*Propriétés physicochimiques*

Point de fusion: 200°C (chlorure de méthylène).
Spectre IR: bandes ν (C=O) à 1685 et 1645 $cm^{-1}$.
Spectre de RMN à 250 MHz dans le DMSO: 10,60 ppm, multiplet, 1 proton, NH; 7,74 ppm, singulet, 2 protons, H aromatiques phtaloyle; 3,55 ppm, multiplet, 2 protons, $CH_2$; 2,62 ppm, singulet, 3 protons, $CH_3$ éthylidénylique; 1,25 ppm, triplet, 3 protons, $CH_3$ éthylique.

*EXEMPLE XIII*

(Isobutylamino-1 éthylidényl)-2 indanedione-1,3; produit de formule (I) dans laquelle $R_1$ = $R_3$ = H, $R_2$ = $CH_2C(CH_3)_2$, $R_4$ = $CH_3$; nom de code COR4008.

*Préparation*

A une solution de 0,3 ml d'acide formique, 20 ml d'isobutylamine, chauffée à 90°C, on ajoute très lentement une solution de 1,5 g d'acétyl-2 indanedione-1,3 dans 150 ml d'éthanol. Le chauffage est poursuivi pendant 2 jours. Le solvant est éliminé par évaporation. Le résidu est purifié par passage sur colonne de gel de silice en éluant par du chlorure de méthylène. Le produit est recristallisé dans l'éther isopropylique. Le rendement est de 81%.

*Propriétés physicochimiques*

Point de fusion: 96,5°C (éther isopropylique).
Spectre IR: bandes ν C = O à 1690 et 1650 $cm^{-1}$.
Spectre de RMN dans $CDCl_3$: 11,03 ppm, multiplet, 1 proton, NH; 7,67 ppm, multiplet, 4 protons, H aromatiques phtaloyle; 3,27 ppm, multiplet, 2 protons, $CH_2$; 2,67 ppm, singulet, 3 protons, $CH_3$ éthylidénylique; 2,00 ppm, multiplet, 1 proton, CH; 1,07 ppm, 2 singulets, 6 protons, 2 $CH_3$.

*EXEMPLE XIV*

(Méthoxy-2 éthyl amino-1 éthylidényl)-2 indanedione-1,3; produit de formule (I) dans laquelle $R_1$ = $R_3$ = H, $R_2$ = $C_2H_4OCH_3$, $R_4$ = $CH_3$; nom de code COR4009.

*Préparation*

A une solution de 0,3 ml d'acide formique, 20 ml de méthoxy-2 éthylamine, chauffée à 90°C, on ajoute très lentement une solution de 1,5 g d'acétyl-2 indanedione-1,3 dans 150 ml d'éthanol. Le chauffage est poursuivi pendant 2 jours. Le solvant est éliminé par évaporation. Le résidu est purifié par passage sur colonne de gel de silice en éluant par du chlorure de méthylène. Le produit est recristallisé dans un mélange éther isopropylique - éthanol. Le rendement est de 77%.

*Propriétés physicochimiques*

Point de fusion: 116,5°C (mélange éther isopropylique - éthanol).
Spectre IR: bandes ν C = O à 1685 et 1640 $cm^{-1}$.
Spectre de RMN dans $CDCl_3$: 11,03 ppm, multiplet, 1 proton, NH; 7,71 ppm, multiplet, 4 protons, H aromatiques phtaloyle; 3,62 ppm, multiplet, 4 protons, 2 $CH_2$; 3,47 ppm, singulet, 3 protons, $OCH_3$; 2,68 ppm, singulet, 3 protons, $CCH_3$.

*EXEMPLE XV*

(Butylamino-1 éthylidényl)-2 indanedione-1,3; produit de formule (I) dans laquelle $R_1$ = $R_3$ = H, $R_2$ = $(CH_2)_3CH_3$, $R_4$ = $CH_3$; nom de code COR4010.

*Préparation*

A une solution de 0,3 ml d'acide formique, 20 ml d'éthanol et 2 ml de butylamine, chauffée à 90°C, on ajoute très lentement une solution de 1,5 g d'acétyl-2 indanedione-1,3 dans 150 ml d'éthanol. Le chauffage est poursuivi pendant 2 jours. Le solvant est éliminé par évaporation. Le résidu est purifié par passage sur colonne de gel de silice en éluant par du chlorure de méthylène. Le produit est recristallisé dans l'éther isopropylique. Le rendement est de 82%.

*Propriétés physicochimiques*

Point de fusion: 66°C (éther isopropylique).
Spectre IR: bandes ν C = O à 1670 et 1645 $cm^{-1}$.
Spectre de RMN dans $CDCl_3$: 10,87 ppm, multiplet, 1 proton, NH; 7,68 ppm, multiplet, 4 protons, H aromatiques phtaloyle; 3,43 ppm, multiplet, 2 protons, $NCH_2$; 2,65 ppm, singulet, 3 protons, $CH_3$ éthylidénylique; 1,63 ppm, multiplet, 4 protons, 2 $CH_2$; 1,00 ppm, multiplet, 3 protons, $CH_3$ butylique.

*EXEMPLE XVI*

(Cyclopropylméthylamino-1 éthylidényl)-2 indanedione-1,3; produit de formule (I) dans laquelle $R_1$ = $R_3$ = H, $R_2$ = $CH_2CH(CH_2)_2$, $R_4$ = $CH_3$; nom de code COR4011.

*Préparation*

A une solution de 0,3 ml d'acide formique, 40 ml d'éthanol et 2 ml d'aminométhylcyclopropane, chauffée à 80-90°C, on ajoute lentement une solution de 1,7 g d'acétyl-2 indanedione-1,3 dans l'éthanol. Le chauffage est poursuivi pendant 2 jours. Le solvant est éliminé par évaporation. Le résidu purifié par passage sur colonne de gel de silice en éluant par du chlorure de méthylène. Le produit est recristallisé dans l'éther isopropylique. Le rendement est de 74%.

*Propriétés physicochimiques*

Point de fusion: 115,5°C (éther isopropylique).

Spectre IR: bandes ν C = O à 1680 et 1645 $cm^{-1}$.

Spectre de RMN dans $CDCl_3$: 10,92 ppm, multiplet, 1 proton, NH; 7,67 ppm, multiplet, 4 protons, H aromatiques phtaloyle; 3,32 ppm, triplet, 2 protons, $NCH_2$; 2,65 ppm, singulet, 3 protons, $CH_3$ éthylidénylique; 1,33-0,3 ppm, multiplet, 5 protons, H cyclopropyliques.

*EXEMPLE XVII*

(Cyclopentylamino-1 éthylidényl)-2 indanedione-1,3; produit de formule (I) dans laquelle $R_1$ = $R_3$ = H, $R_2$ = $CH(CH_2)_4$, $R_4$ = $CH_3$; nom de code COR4012.

*Préparation*

A une solution de 0,3 ml d'acide formique, 40 ml de cyclopentylamine, chauffée à 80-90°C, on ajoute lentement une solution de 1,7 g d'acétyl-2 indanedione-1,3 dans l'éthanol. Le chauffage est poursuivi pendant 1 jour 1/2. Le solvant est éliminé par évaporation. Le résidu est purifié par passage sur colonne de gel de silice en éluant par du chlorure de méthylène. Le produit est recristallisé dans l'éther isopropylique. Le rendement est de 88%.

*Propriétés physicochimiques*

Point de fusion: 112°C (éther isopropylique).

Spectre IR: bandes ν C = O à 1680 et 1645 $cm^{-1}$.

Spectre de RMN dans $CDCl_3$: 10,98 ppm, multiplet, 1 proton, NH; 7,67 ppm, multiplet, 4 protons, H aromatiques phtaloyle; 4,1 ppm, multiplet, 1 proton, CH; 2,67 ppm, singulet, 3 protons, $CH_3$ éthylidénylique; 2,23 - 1,47 ppm, multiplet, 8 protons, 4 $CH_2$ cycliques.

*EXEMPLE XVIII*

(Cyclohexylamino-1 éthylidényl)-2 indanedione-1,3; produit de formule (I) dans laquelle $R_1$ = $R_3$ = H, $R_2$ = $CH(CH_2)_5$, $R_4$ = $CH_3$; nom de code COR4013.

*Préparation*

A une solution de 0,3 ml d'acide formique, 40 ml d'éthanol et 2 ml de cyclohexylamine, chauffée à 85°C, on ajoute lentement une solution de 1,6 g d'acétyl-2 indanedione-1,3 dans l'éthanol. Le chauffage est poursuivi pendant 1 jour 1/2. Le solvant est éliminé par évaporation. Le résidu est purifié par passage sur colonne de gel de silice en éluant par du chlorure de méthylène. Le produit est recristallisé dans l'éther isopropylique. Le rendement est de 89%.

*Propriétés physicochimiques*

Point de fusion: 117°C (éther isopropylique).

Spectre IR: bandes ν C = O à 1680 et 1640 $cm^{-1}$.

Spectre de RMN dans $CDCl_3$: 11,02 ppm, multiplet, 1 proton, NH; 7,65 ppm, multiplet, 4 protons, H aromatiques phtaloyle; 3,67 ppm, multiplet, 1 proton, CH; 2,68 ppm, singulet, 3 protons, $CH_3$ éthylidénylique; 2,2 - 1,17 ppm, multiplet, 10 protons, 5 $CH_2$ cycliques.

*EXEMPLE XIX*

(Cyclopropylamino-1 éthylidényl)-2 indanedione-1,3; produit de formule (I) dans laquelle $R_1$ = $R_3$ = H, $R_2$ = $CH(CH_2)_2$, $R_4$ = $CH_3$; nom de code COR4014.

*Préparation*

A une solution de 0,3 ml d'acide formique, 40 ml d'éthano et 2 ml de cyclopropylamine, chauffée à 85°C, on ajoute lentement une solution de 1,6 g d'acétyl-2 indanedione-1,3 dans l'éthanol. Le chauffage est poursuivi pendant 1 jour. Le solvant est éliminé par évaporation. Le résidu est purifié par passage sur colonne de gel de silice en éluant par du chlorure de méthylène. Le produit est recristallisé dans l'éther isopropylique. Le rendement est de 83%.

*Propriétés physicochimiques*

Point de fusion: 96,5°C (éther isopropylique).

Spectre IR: bandes ν C = O à 1695 et 1645 $cm^{-1}$.

Spectre de RMN dans $CDCl_3$: 10,73 ppm, multiplet, 1 proton, NH; 7,65 ppm, multiplet, 4 protons, H aromatiques phtaloyle; 2,67 ppm, multiplet, 1 proton, CH; 2,67 ppm, singulet, 3 protons, $CH_3$ éthylidénylique; 1,03 - 0,73 ppm, multiplet, 4 protons, 2 $CH_2$ cycliques.

*EXEMPLE XX*

N-acétylsulfonamido-6 chloro-5 (isopropylamino-1 éthylidényl)-2 indanedione-1,3; produit de formule (I) dans laquelle $R_1$ = H, $R_2$ = $CH(CH_3)_2$, $R_3$ = $SO_2NHCOCH_3$ en 6 et Cl en 5 et $R_4$ = $CH_3$; nom de code COR4015.

*Préparation*

A une solution de 0,3 ml d'acide formique, 2 ml d'isopropylamino et 40 ml d'éthanol, chauffée à 90°C, on ajoute lentement une solution de 1,25 g d'acétyl-2 N-acétylsulfonamido-6 chlor-5 indanedione-1,3 dans l'éthanol. Le chauffage est poursuivi pendant 2 jours. Le solvant est éliminé par évaporation. Le résidu est purifié par passage sur colonne de gel de silice en éluant par un mélange du chlorure de méthylène - éthanol (9/1). Le produit est recristallisé dans l'éthanol. Le rendement est de 81%.

*Propriétés physicochimiques*

Point de fusion: 220°C (chlorure de méthylène - éthanol).

Spectre IR: bandes ν C = O à 1740, 1690, 1660 et 1645 $cm^{-1}$; bandes ν NH à 3235 et 3255 $cm^{-1}$.

Spectre de RMN dans $CDCl_3$: 10,92 ppm, multiplet, 2 protons, NH; 8,37 ppm, doublet, 1 proton, H aromatique phtaloyle; 7,8 ppm, doublet, 1 proton,

H aromatique phtaloyle; 4,12 ppm, multiplet, 1 proton, CH; 2,67 ppm, singulet, 3 protons, $CH_3$ éthylidénylique; 2,17 ppm, singulet, 3 protons, $COCH_3$; 1,42 ppm, doublet, 6 protons, 2 $CCH_3$ isopropyliques.

*EXEMPLE XXI*

Dichloro-5,6 (isopropylamino-1 éthylidényl)-2 indanedione-1,3; produit de formule (I) dans laquelle $R_1$ = H, $R_2$ = $CH(CH_3)_2$, $R_3$ = 2 Cl en 5 et en 6 et $R_4$ = $CH_3$; nom de code COR4016.

*Préparation*

A une solution de 0,5 ml d'acide formique, 3 ml d'isopropylamine et 40 ml d'éthanol, chauffé à 90°C, on ajoute lentement une solution de 2 g d'acétyl-2 dichloro-5,6 indanedione-1,3 dans l'éthanol. Le chauffage est poursuivi pendant 2 jours. Le solvant est éliminé par évaporation. Le résidu est purifié par passage sur colonne de gel de silice en éluant par du chlorure de méthylène. Le produit est recristallisé dans l'éthanol. Le rendement est de 86%.

*Propriétés physicochimiques*

Point de fusion: 204°C (éthanol).

Spectre IR: bandes ν C = O à 1685 et 1640 $cm^{-1}$.

Spectre de RMN dans $CDCl_3$: 10,87 ppm, multiplet, 1 proton, NH; 7,77 ppm, multiplet, 2 protons, H aromatiques phtaloyle; 4,01 ppm, multiplet, 1 proton, CH; 2,78 ppm, singulet, 3 protons, $CH_3$ éthylidénylique; 1,44 ppm, doublet, 6 protons, 2 $CH_3$ isopropyliques.

*EXEMPLE XXII*

(Méthylamino-1 butylidényl)-2 indanedione-1,3; produit de formule (I) dans laquelle $R_1$ = H, $R_2$ = $CH_3$, $R_3$ = H et $R_4$ = $C_3H_7$; nom de code COR4017.

*Préparation*

A une solution constituée par 0,4 ml d'acide formique, 5 ml de solution benzénique de méthylamine à 10% et 40 ml d'éthanol, chauffée à 90°C, on ajoute très lentement une solution éthanolique de 2,5 g de propionyl-2 indanedione-1,3. Le chauffage du mélange est poursuivi pendant 1 jour. Le solvant est éliminé par évaporation. Le résidu est purifié par passage sur colonne de gel de silice en éluant par du chlorure de méthylène. Le rendement est de 51%.

*Propriétés physicochimiques*

Point de fusion: 139°C (chlorure de méthylène).

Spectre IR: bandes ν C = O à 1685 et 1645 $cm^{-1}$.

Spectre de RMN dans $CDCl_3$: 10,62 ppm, multiplet, 1 proton, NH; 7,57 ppm, multiplet, 4 protons, H aromatiques phtaloyle; 3,1 ppm, doublet, 3 protons, $NCH_3$; 3,03 ppm, multiplet, 2 protons, $CH_2$ C=C; 2,13 à 1,38 ppm, multiplet, 2 protons, C-$CH_2$-C; 1,1 ppm, multiplet, 3 protons, $CCH_3$.

*EXEMPLE XXIII*

[(Dichloro-3,4 benzylamino)-1 éthylidényl)-2 indanedione-1,3; produit de formule (I) dans laquelle $R_1$ = $R_3$ = H, $R_2$ = $CH_2$- 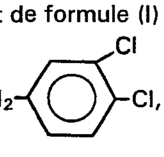 , $R_4$ = $CH_3$; nom de code COR4018.

*Préparation*

A une solution de 0,3 ml d'acide formique, 40 ml d'éthanol et 2 ml de dichloro-3,4 benzylamine, chauffée à 90°C, on ajoute lentement une solution de 1,25 g d'acétyl-2 indanedione-1,3 dans 200 ml d'éthanol. Le chauffage est poursuivi pendant une journée. Le solvant est éliminé par évaporation. Le produit est recristallisé dans l'éthanol. Le rendement est de 78%.

*Propriétés physicochimiques*

Point de fusion: 154°C (éthanol).

Spectre IR: bandes ν C = O à 1680 et 1645 $cm^{-1}$.

Spectre de RMN dans $CDCl_3$: 10,97 ppm, multiplet, 1 proton, NH; 7,53 ppm, multiplet, 4 protons, H aromatiques phtaloyle; 7,3 à 6,93 ppm, multiplet, 3 protons, H aromatiques benzéniques; 4,5 ppm, doublet, 2 protons, $CH_2$; 2,6 ppm, singulet, 3 protons, $CH_3$.

*EXEMPLE XXIV*

(Méthylamino-1 isobutylidényl)-2 indanedione-1,3; produit de formule (I) dans laquelle $R_1$ = H, $R_2$ = $CH_3$, $R_3$ = H et $R_4$ = $CH(CH_3)_2$; nom de code COR4019.

*Préparation*

A une solution constituée par 0,5 ml d'acide formique, 8 ml de solution alcoolique de méthylamine à 33% et 40 ml d'éthanol, chauffée à 90°C, on ajoute très lentement une solution de 5,9 g d'isobutyryl-2 indanedione-1,3 dans 150 ml d'éthanol. Le chauffage du mélange réactionnel est poursuivi pendant 1 jour et demi. Le solvant est éliminé par évaporation. Le résidu est purifié par passage sur colonne de gel de silice en éluant par de l'éther éthylique. Le produit obtenu est ensuite recristallisé dans l'éther isopropylique. Le rendement est de 43%.

*Propriétés physicochimiques*

Point de fusion: 117,5°C (éther isopropylique).

Spectre IR: bandes ν C = O à 1675 et 1635 $cm^{-1}$.

Spectre de RMN dans $CDCl_3$: 11,37 ppm, multiplet, 1 proton, NH; 7,63 ppm, multiplet, 4 protons, H aromatiques phtaloyle; 4,37 ppm, multiplet, 1 proton, CH; 3,23 ppm, doublet, 3 protons, $NCH_3$; 1,43 ppm, doublet, 6 protons, 2 $CH_3$ isopropyliques.

*EXEMPLE XXV*

(Ethylamino-1 propylidényl)-2 indanedione-1,3; produit de formule (I) dans laquelle $R_1$ = H, $R_2$ = $C_2H_5$, $R_3$ = H et $R_4$ = $C_2H_5$; nom de code COR4021.

*Préparation*

A une solution constituée par 0,2 ml d'acide formique, 3 ml de solution aqueuse d'éthylamine à 70% et 25 ml d'éthanol, chauffée à 80-90°C, on ajoute lentement une solution de 1,6 g de propionyl-2 indanedione-1,3 dans l'éthanol. Le chauffage du mélange réactionnel est poursuivi pendant 4 heures, puis le mélange est maintenu à température ambiante pendant une nuit. Le solvant est éliminé par évaporation. Le résidu est purifié par passage sur colonne de gel de silice en éluant par du chlorure de méthylène.

Le produit obtenu est recristallisé dans un mélange éther isopropylique - éthanol (5/1). Le rendement est de 94%.

*Propriétés physicochimiques*

Point de fusion: 106°C (éther isopropylique - éthanol).

Spectre IR: bandes ν C = O à 1690 et 1645 $cm^{-1}$.

Spectre de RMN dans $CDCl_3$: 10,62 ppm, multiplet, 1 proton, NH; 7,55 ppm, multiplet, 4 protons, H aromatiques phtaloyle; 4,53 ppm, quadruplet, 2 protons, $CH_2$; 1,37 ppm, triplet, 3 protons, N-C-$CH_3$; 1,25 ppm, triplet, 3 protons, C-C-$CH_3$.

*EXEMPLE XXVI*

(Isopropylamino-1 butylidényl)-2 indanedione-1,3; produit de formule (I) dans laquelle $R_1$ = H, $R_2$ = $CH(CH_3)_2$, $R_3$ = H et $R_4$ = $C_3H_7$; nom de code COR4022.

*Préparation*

A une solution de 0,3 ml d'acide formique, 2,5 ml d'isopropylamine et 40 ml d'éthanol, chauffée à 90°C, on ajoute lentement une solution éthanolique de 2,4 g de propionyl-2 indanedione-1,3. Le chauffage du mélange réactionnel est poursuivi pendant 2 jous. Le solvant est éliminé par évaporation. Le résidu est purifié par deux passage successifs sur colonne de gel de silice en éluant par du chlorure de méthylène puis par de l'éther isopropylique. Le produit cristallise lentement dans un peu d'éther de pétrole. Le rendement est de 31%.

*Propriétés physicochimiques*

Point de fusion: 70,5°C (éther de pétrole).

Spectre IR: bandes ν C = O à 1680 et 1645 $cm^{-1}$.

Spectre de RMN dans $CDCl_3$: 10,82 ppm, multiplet, 1 proton, NH; 7,60 ppm, multiplet, 4 protons, H aromatiques phtaloyle; 4,4 - 3,6 ppm, multiplet, 1 proton, CH; 3,07 ppm, multiplet, 2 protons, $C=CCH_2$; 2,06 à 0,83 ppm, multiplet, 5 protons, $CH_2$ + $CH_3$; 1,37 ppm, doublet, 6 protons, 2 $CH_3$ isopropyliques.

*EXEMPLE XXVII*

(Isopropylamino-1 isobutylidényl)-2 indanedione-1,3; produit de formule (I) dans laquelle $R_1$ = H, $R_2$ = $CH(CH_3)_2$, $R_3$ = H et $R_4$ = $CH(CH_3)_2$; nom de code COR4023.

*Préparation*

A une solution de 0,5 ml d'acide formique, 5 ml d'isopropylamine et 40 ml d'éthanol, chauffée à 100°C, on ajoute lentement une solution de 2,8 g d'isobutyryl-2 indanedione-1,3 dans l'éthanol. Le chauffage du mélange réactionnel est poursuivi pendant 2 jours. Le mélange réactionnel est transvasé en réacteur et chauffé pendant 4 heures à 80°C puis pendant 3 heures à 100°C. Le solvant est éliminé par évaporation. Le résidu est purifié par passage sur colonne de gel de silice en éluant par du chlorure de méthylène. Le produit est recristallisé dans un mélange isopropylique (1/3)- éther de pétrole (2/3). Le rendement est de 20,3%.

*Propriétés physicochimiques*

Point de fusion: 100°C (éther isopropylique - éther de pétrole).

Spectre IR: bandes ν C = O à 1690 et 1645 $cm^{-1}$.

Spectre de RMN dans $CDCl_3$: 11,47 ppm, multiplet, 1 proton, NH; 7,63 ppm, multiplet, 4 protons, H aromatiques phtaloyle; 4,58 à 3,83 ppm, multiplet, 2 protons, 2 CH; 1,5 ppm, doublet, 6 protons, 2 $CH_3$ N-isopropyliques; 1,35 ppm, doublet, 6 protons, 2 $CH_3$ C-isopropyliques.

*EXEMPLE XXVIII*

(Isopropylamino-1 propylidényl)-2 indanedione-1,3; produit de formule (I) dans laquelle $R_1$ = H, $R_2$ = $CH(CH_3)_2$, $R_3$ = H et $R_4$ = $C_2H_5$; nom de code COR4025.

*Préparation*

A une solution de 0,3 ml d'acide formique, 5 ml d'isopropylamine et 40 ml d'éthanol, chauffée à 100°C, on ajoute lentement une solution de 1,7 g de propionyl-2 indanedione-1,3 dans 200 ml d'éthanol. Le chauffage du mélange réactionnel est poursuivi pendant 2 jours. Le solvant est éliminé par évaporation. Le résidu est purifié par passage sur colonne de gel de silice en éluant par du chlorure de méthylène. Le produit obtenu cristallise lentement dans l'éther de pétrole. Le rendement est de 85%.

*Propriétés physicochimiques*

Point de fusion: 69°C (éther de pétrole).

Spectre IR: bandes ν C = O à 1685 et 1640 $cm^{-1}$.

Spectre de RMN dans $CDCl_3$: 10,83 ppm, multiplet, 1 proton, NH; 7,67 ppm, multiplet, 4 protons, H aromatiques phtaloyle; 4,33 - 3,77 ppm, multiplet, 1 proton, CH; 3,1 ppm, quadruplet, 2 protons $CH_2$; 1,38 ppm, doublet, 6 protons, 2 $CH_3$ isopropyliques; 1,32 ppm, triplet, 3 protons, $CH_3$.

*EXEMPLE XXIX*

(Amino-1 éthylidényl)-2 indanedione-1,3; produit de formule (I) dans laquelle $R_1$ = $R_2$ = $R_3$ = H, $R_4$ = $CH_3$; nom de code COR4026.

*Préparation*

Le mélange constitué par 100 ml d'éthanol saturé en ammoniac et 1,48 g d'acétyl-2 indanedione-1,3 est chauffé pendant 4 heures à 80°C en tube scellé. Le solvant est éliminé par évaporation. Le résidu est purifié par passage sur colonne de gel de silice en éluant par de l'éther éthylique. Le rendement est de 71%.

*Propriétés physicochimiques*

Point de fusion: 226°C (éther éthylique).

Spectre IR: bandes ν C = O à 1690 et 1640 $cm^{-1}$, épaulement à 1650 $cm^{-1}$; bandes ν $NH_2$ à 3200 et 3315 $cm^{-1}$.

Spectre de RMN dans DMSO $D_6$: 9,81 à 8,88 ppm, multiplet, 2 protons, $NH_2$; 7,58 ppm, multiplet, 4 protons, H aromatiques phtaloyle; 2,46 ppm, singulet, 3 protons, $CH_3$.

*EXEMPLE XXX*

(Cyclopropylméthylamino-1 propylidényl)-2 indanedione-1,3; produit de formule (I) dans laquelle $R_1$ = $R_3$ = H, $R_2$ = $CH_2CH(CH_2)_2$, $R_4$ = $C_2H_5$; nom de code COR4027.

*Préparation*

A une solution de 0,3 ml d'acide formique, 40 ml d'éthanol et 2 ml d'aminométhylcyclopropane, chauffée à 80°C, on ajoute lentement une solution de 1,5 g de propionyl-2 indanedione-1,3 dans 100 ml d'éthanol. Le chauffage est poursuivi pendant 2 jours à 80-90°C. Le solvant est éliminé par évaporation. Le résidu est purifié par passage sur colonne de gel de silice en éluant par de l'éther éthylique. Le produit est recristallisé dans un mélange éther isopropylique - éthanol. Le rendement est de 50%.

*Propriétés physicochimiques*

Point de fusion: 94°C (éther isopropylique - éthanol).

Spectre IR: bandes ν C = O à 1690 et 1645 $cm^{-1}$.

Spectre de RMN dans $CDCl_3$: 10,68 ppm, multiplet, 1 proton, NH; 7,48 ppm, multiplet, 4 protons, H aromatiques phtaloyle; 3,26 ppm, multiplet, 2 protons, $NCH_2$; 3,01 ppm, quadruplet, 2 protons, $CH_2$; 1,23 ppm, multiplet, 4 protons, $CH_3$ éthylidénylique + CH cyclique; 0,83 - 0,21 ppm, multiplet, 4 protons, 2 $CH_2$ cycliques.

*EXEMPLE XXXI*

(Amino-1 propylidényl)-2 indanedione-1,3; produit de formule (I) dans laquelle $R_1$ = $R_2$ = $R_3$ = H, $R_4$ = $C_2H_5$; nom de code COR4028.

*Préparation*

Le mélange constitué par 150 ml d'éthanol en ammoniac et 1,7 g de propionyl-2 indanedione-1,3 est chauffé pendant 4 heures à 80°C en tube scellé. Le solvant est éliminé par évaporation. Le résidu est purifié par passage sur colonne de gel de silice en éluant par de l'éther isopropylique. Le rendement est de 62,7%.

*Propriétés physicochimiques*

Point de fusion: 139°C (éther isopropylique).

Spectre IR: bandes ν C = O à 1690 et 1640 $cm^{-1}$; bandes ν $NH_2$ à 3215 et 3365 $cm^{-1}$.

Spectre de RMN dans $CDCl_3$: 10 ppm, multiplet, 1 proton, NH; 7,7 ppm, multiplet, 4 protons, H aromatiques phtaloyle; 6,40 ppm, multiplet, 1 proton, NH; 3,07 ppm, quintuplet, 2 protons, $CH_2$; 1,32 ppm, triplet, 3 protons $CH_3$.

*EXEMPLE XXXII*

(Butylamino-1 propylidényl)-2 indanedione-1,3; produit de formule (I) dans laquelle $R_1$ = $R_3$ = H, $R_2$ = $(CH_2)_3CH_3$, $R_4$ = $C_2H_5$; nom de code COR4029.

*Préparation*

A une solution de 0,3 ml d'acide formique, 40 ml d'éthanol et 4 ml de butylamine, chauffée à 90°C, on ajoute lentement une solution de 1,6 g de propionyl-2 indanedione-1,3 dans 150 ml d'éthanol. Le chauffage est poursuivi pendant 1 jour. Le solvant est éliminé par évaporation. Le résidu est purifié par passage sur colonne de gel de silice en éluant par du chlorure de méthylène. Le produit est recristallisé dans un mélange éther isoproylique - éther de pétrole. Le rendement est de 52%.

*Propriétés physicochimiques*

Point de fusion: 72°C (éther isopropylique - éther de pétrole).

Spectre IR: bandes ν C = O à 1685 et 1645 $cm^{-1}$.

Spectre de RMN dans $CDCl_3$: 10,83 ppm, multiplet, 1 proton, NH; 7,67 ppm, multiplet, 4 protons, H aromatiques phtaloyle; 3,47 ppm, quadruplet, 2 protons, N-$CH_2$; 3,1 ppm, quadruplet, 2 protons, C=C-$CH_2$; 2,03 à 0,78 ppm, multiplet, 10 protons, C-$CH_2CH_2$-C et 2 $CH_3$.

*EXEMPLE XXXIII*

[(Méthoxy-2 éthyl) amino-1 propylidényl)-2 indanedione-1,3; produit de formule (I) dans laquelle $R_1$ = $R_3$ = H, $R_2$ = $C_2H_4OCH_3$, $R_4$ = $C_2H_5$; nom de code COR4030.

*Préparation*

A une solution de 0,3 ml d'acide formique, 40 ml d'éthanol et 4 ml de méthoxy-2 éthylamine, chauffée à 90°C, on ajoute très lentement une solution de 1,6 g de propionyl-2 indanedione-1,3 dans 150 ml d'éthanol. Le chauffage est poursuivi pendant un jour et demi. Le solvant est éliminé par évaporation. Le résidu est purifié par passage sur colonne de gel de silice en éluant par du chlorure de méthylène. Le produit est recristallisé à froid dans de l'éther de pétrole. Le rendement est de 84%.

*Propriétés physicochimiques*

Point de fusion: 64°C (éther de pétrole).

Spectre IR: bandes ν C = O à 1700 et 1650 $cm^{-1}$.

Spectre de RMN dans $CDCl_3$: 10,93 ppm, multiplet, 1 proton, NH; 7,63 ppm, multiplet, 4 protons, H aromatiques phtaloyle; 3,63 ppm, multiplet, 4 protons, $NCH_2$ et $OCH_2$; 3,45 ppm, singulet, 3 protons, $OCH_3$; 3,1 ppm, quadruplet, 2 protons, $CH_2$; 1,25 ppm, triplet, 3 protons, $CCH_3$.

Les résultats d'études toxicopharmacologiques réalisées sur les produits selon la présente invention sont exposés ci-après.

*TOXICITÉ*

Administrés à la souris par voie orale à la dose de 300 mg(kg ou par voie intrapéritonéale à la dose de 200 mg/kg, les COR3760, COR3773, COR3774, COR3789, COR3792, COR4003, COR4004, COR4006, COR4007, COR4008, COR4009, COR4010, COR4011, COR4012, COR4013, COR4014, COR4015, COR4016 n'induisent aucune mortalité. Les COR3788, COR4001 et COR4002 induisent 100% de mortalité à ces doses et par ces voies d'administration, mais n'induisent auncune mortalité quand ils sont administrés par voie intrapéritonéale à la dose de 100 mg/kg; les COR4001 et COR4002 n'induisent aucune mortalité quand ils sont administrés par voie orale à la dose de 100 mg/kg. Le COR3760 administré par voie orale à

la souris, en solution dans le Tween 20% présente une DL50 de 736(594-911) mg/kg.

*PHARMACOLOGIE*

L'activité diurétique a été déterminée sur plusieurs modèles expérimentaux. Chez le rat soumis à une surcharge hydrique, administrés par voie orale à la dose de 20 mg/kg, le COR3760, le COR3789, le COR3792, le COR4002, le COR4003, le COR4009, le COR4010, le COR4012 et le COR4014 multiplient l'excrétion urinaire du sodium par respectivement un facteur 2,9 - 3,1 - 5,9 - 2,6 - 7,2 - 3,5 - 3,8 - 2,9 - 2,4. Administrés à la dose de 10 mg/kg, dans les mêmes conditions, les COR3760, COR3789, COR3792, COR4003 et COR4010 multiplient l'excrétion sodique par respectivement 2,3 - 2,9 - 2,8 - 2,4 et 2,5. La spironolactone multiplie quant à elle l'excrétion sodique d'un facteur 3,6 à la dose de 20 mg/kg dans les mêmes conditions, le COR3792 multiplie l'excrétion sodique par 3,3. Administré par voie orale chez le rat ou la souris soumis à une surcharge saline, le COR3760 augmente l'excrétion urinaire volumétrique mesurée à la sixième heure de respectivement 49, 119 et 134% chez la souris et 28, 84 et 116% chez le rat après administration de 10, 20, et 40 mg/kg. Le COR3760 a été administré par voie orale au rat soumis à une surcharge hydrique et l'excrétion urinaire volumétrique, le sodium et le potassium excrétés ont été mesurés sur une période de 6 heures; ces paramètres ont varié de respecitvement +17%, +102%, +165% à 10 mg/kg, +19%, +147%, +180% à 20 mg/kg et +36%, +415% et +239% à 40 mg/kg; le rapport Na/K passe de 0,56 chez les animaux témoins à 0,4 à 10 mg/kg, 0,49 à 20 mg/kg et 0,8 à 40 mg/kg. Au cours d'une seconde expérimentation, le COR3760 a entrainé des variations de +19%, +157%, +70% à 40 mg/kg, +19%, +319%, +90% à 60 mg/kg et +29%, +472% et +77% à 80 mg/kg; le rapport Na/K passe de 0,59 chez les animaux témoins à 0,88 à 40, 1,15 à 60 et 1,88 à 80 mg/kg.

L'activité diurétique des produits selon l'invention a été également déterminée chez le rat Wistar soumis à une surcharge saline selon une modification du test décrit par Wiebelhaus et coll., Fed. Proc., 1960, 19, 364, en procédant à une sélection des animaux. A la dose de 20 mg/kg, les COR4003, COR4008, COR4009, COR4010, COR4011, COR4012, COR4014, COR4016, COR4021, COR4025, augmentent l'excrétion urinaire volumétrique mesurée au bout de 6 heures de respecitvement 56, 54, 65, 95, 73, 54, 33, 40, 99 et 92%. L'excrétion urinaire du sodium augmente de respectivement 56, 24, 42, 59, 43, 33, 29, 43, 31, 83, 26, 99, 40% toujours à cette dose pour les COR4003, COR4008, COR4009, COR4010, COR4011, COR4012, COR4014, COR4016, COR4017, COR4021, COR4023, COR4025 et COR4026. Le rapport Na/K demeure la plupart du temps inchangé. Testé dans les mêmes conditions le furosémide, administré à la dose de 20 mg/kg entraine 63% d'augmentation de l'excrétion urinaire volumétrique et 53% d'augmentation de l'excrétion urinaire du sodium.

L'activité antihypertensive a été chez le rat vigile spontanément hypertendu. La pression artérielle est mesurée de façon indirecte sur la queu 2, 4, 6 heures après l'administration par voie orale du produit à tester. Pour le COR4009, administré à la dose de 100 mg/kg, la réduction de pression est respectivement de 8, 13, 13% à ces échéances.

L'activité spasmolytique a été déterminée in vitro par la mesure de l'effet du produit à tester sur les contractions de l'iléon de cobaye induites par stimulation transmurale dans une solution de Krebs oxygénée à 32°C. Le COR4010 et le COR3773 utilisés à la concentration de 2 microg/ml entrainent 50% d'inihibition de ces contractions. La papavérine présente la même activité à la même concentration.

L'activité antiinflammatoire a été déterminée chez le rat par la mesure de l'inhibition de l'oedème induit par l'injection intraplantaire de 0,1 ml d'une suspension à 1% de carragénine chez le rat. Les produits à tester sont administrés par voie orale 1H avant l'injection de carragénine et la mesure est réalisée 3h après cette injection. Les COR3789, COR3792, COR4003, COR4008 et COR4010 administrés à 100 mg/kg entrainent respectivement 43, 42, 41, 38 et 39% d'inhibition. Administré à 200 mg/kg, le COR3773, entraine 53% d'inhibition. L'aspirine administrée dans les mêmes conditions à 150 mg/kg entraine 40% d'inhibition de l'oedème.

L'activité bronchodilatatrice a été déterminée in vitro par la mesure du débit d'une solution de Tyrode à travers un poumon de cobaye isolé perfusé, contracté par l'addition au fluide de perfusion de 0,05 microg/ml de méthacholine. Les COR3760, COR3789, COR3792, COR4003, COR4008, COR4010 et COR4014, utilisés respectivement aux concentrations de 50, 50, 100, 10, 50, 50 et 10 microg/ml augmentent ce débit de 50%. L'aminophyline entraine cette même activité à la concentration de 100 microg/ml.

L'activitè anticholinergique a été appréciée par la mesure des contractions induites par 0,1 microg/ml d'acétylcholine sur des segments d'iléon de cobaye isolés. Les COR3760, COR3789, COR3792, COR4003, COR4008, COR4010, COR4014, utilisés respectivement aux concentrations de 50. 25, 25, 50, 25, 10, 25 microg/ml inhibent ces contractions de 80%.

L'activité antidépressive a été dans le test de potentialisation de la toxicité de la yohimbine et dans le test de l'inhibition du ptosis induit par la réserpine.

On injecte par voie sous cutanée à des souris, 1h avant l'administration par voie orale du produit à tester, une dose non léthale de yohimbine soit 15 mg/kg. Le COR3760 administré à la dose de 100 mg/kg entraine 80% de mortalité dans ces conditions.

Des souris reçoivent par voie orale le produit à tester 90 mn avant l'injection par voie intrapéritonéale de 5 mg/kg de réserpine solubilisée. On cote le ptosis selon un index allant de 0 à 6, la cote 0 correspondant à un ptosis maximum et la cote 6 à l'absence de ptosis. Les COR3760 et COR4011 conduisent dans les conditions de l'expérience à un ptosis coté à 4 pour une dose de respectivement 200 et 50 mg/kg.

L'activité anticalcique a été déterminée in vitro sur deux modèles expérimentaux.

Sur oreillette gauche de cobaye stimulée électriquement à 150 battements/mn dans une solution de Tyrode pauvre en calcium (0,6 mM), les COR3789, COR3792, COR4009, COR4010 et COR4011 employés respectivement à 25, 100, 50, 10 et 25 microg/ml inhibent de 50% l'augmentation de la force de contraction induite par l'addition de 0,6 mM de calcium.

Les COR3789, COR4010 et COR4011 employés à la concentration de 10 microg/ml inhibent l'augmentation, induite par le calcium, des contractions spontanées de strips de veines mésentériques porte de rats normotendus.

L'activité antiH2 a été appreciée in vitro dans le test de l'inhibition de l'effet chronotrope induit par 5 microg/ml d'histamine sur des oreillettes droites de cobaye non stimulées; les COR4003 et COR4011, utilisés respectivement à la concentration de 100 et 50 microg/ml, inhibent de 50% cet effet chronotrope tout en ne présentant pas d'effet chronotrope négatif intrinsèque.

L'activité antihistaminique a été appréciée in vitro par l'inhibition de la réponse contractile de segments isolés d'iléon de cobaye induite par 0,5 microg/ml d'histamine. Le COR4003 inhibe cette réponse quand il est utilisé à la concentration de 50 microg/ml.

L'activité anticonvulsivante est appréciée dans le test de la prévention des convulsions induites par des électrochocs d'intensité maximale. Une heure après administration du COR4009, ou de méprobamate par voie orale chez la souris à la dose de 100 mg/kg, on n'observe plus de convulsions après application d'électrochocs.

L'activité anticonvulsivante est également déterminée dans le test des inhibitions des convulsions induites par la bicuculline. Des souris reçoivent par voie intrapéritonéale le produit à tester, puis 30 mn et 5 heures plus tard, deux injections séparées de 0,5 mg de bicuculline. Le COR4009 ou le valproate de sodium, administré à la dose de 100 mg/kg, inhibe les convulsions induites par la première injection, sans inhiber celles induites par la deuxième. Les résultats de ce test sont le reflet d'une activité gabaergique directe.

Compte tenu de leurs activités pharmacologiques jointes à una faible toxicité, les produits faisant l'objet de la présente invention pourront être utilisés en thérapeutique humaine ou vétérinaire.

Associés aux recipients habituels, ils pourront être utilisés par exemple compte tenu de leurs activités diurétiques et antioedémateuses, dans le traitement des oedèmes cardiaques, rénaux ou hépatiques et de l'hypertension artérielle. Compte tenu de leurs activités spasmolytiques, bronchodilatatrices et anticholinergiques, ils pourront être utilisés dans le traitement des manifestations spasmodiques en particulier des voies digestives et respiratoires, dans le traitement des états asthmatiques. Compte tenu de leurs activités psychotropes, ils pourront être utilisés dans le traitement des états dépressifs et des épilepsies. Compte tenu de leurs activités anticalciques ils pourront être utilisés dans le traitement de l'angor. Compte tenu de leurs activités antiH2, ils pourront être utilisés dans le traitement des ulcères gastroduodénaux.

Associés aux excipients habituels, ils seront administrés, selon l'indication, par exemple par voie orale sous forme de dragées, comprimés, sirops, ampoules, par voie rectale sous forme de suppositoires, par voie intramusculaire ou intraveineuse. On peut aussi les administrer, si nécessaire, sous forme d'aérosols. Les doses administrées varieront selon l'indication et le sujet de 1 à 100 mg/kg en 1 à 6 prises pour la voie orale, de 1 à 100 mg/kg en 1 ou 2 prises pour la voie rectale, de 0,5 à 50 mg par injection pour les voies parentérales.

**Revendications**

1. Nouveaux produits chimiques de formule générale

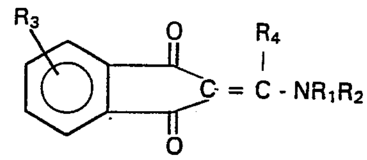

dans laquelle

$R_1$ représente un atome d'hydrogène ou un groupement alkyl inférieur tel que méthyl,

$R_2$ représente un atome d'hydrogène ou un groupement alkyl inférieur de $C_1$ à $C_4$ linéaire ou ramifié choisi dans le groupe formé par méthyl, éthyl, propyl, isopropyl, butyl, isobutyl, tertiobutyl, ou bien un groupement alcoxyéthyl, de préférence méthoxyéthyl, ou bien un groupement cycloalkyl choisi dans le groupe formé par cyclopropyl, cyclobutyl, cyclopentyl, cyclohexyl et cycloheptyl, ou bien un groupement cycloalkylméthyl, de préférence cyclopropylméthyl, ou bien un groupement benzyl substitué ou non sur le cycle aromatique par un ou plusieurs substituants choisis parmi chloro, bromo, fluoro, alkyl inférieur de $C_1$ à $C_4$, alcoxy inférieur de $C_1$ à $C_4$, trifluorométhyl,

$R_3$ représente un atome d'hydrogène ou un ou plusieurs substituants, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl.

2. Procédé de préparation des produits selon la revendication 1 tels que $NR_1R_2$ soit différent de $NH_2$ caractérisé en ce que l'on fait réagir une indanedione-1,3 de formule générale

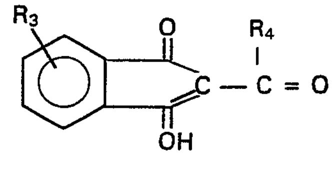

dans laquelle

$R_3$ représente un atome d'hydrogène ou un ou plusieurs substituants, identiques ou différents, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl,

et l'amine de formule générale $HNR_1R_2$ dans laquelle $R_1$ représente un atome d'hydrogène ou un groupement alkyl inférieur tel que méthyl,

$R_2$ représente un groupement alkyl inférieur de $C_1$ à $C_4$ linéaire ou ramifié choisi dans le groupe formé par méthyl, éthyl, propyl, isopropyl, butyl, isobutyl, ou bien un groupement alcoxyéthyl, de préférence méthoxyéthyl, ou bien un groupement cycloalkyl choisi dans le groupe formé par cyclopropyl, cyclobutyl, cyclopentyl, cyclohexyl et cycloheptyl, ou bien un groupement cycloalkylméthyl, de préférence cyclopropylméthyl, ou bien un groupement benzyl substitué ou non sur le cycle aromatique par un ou plusieurs substituants choisis parmi chloro, bromo, fluoro, alkyl inférieur de $C_1$ à $C_4$, alcoxy inférieur de $C_1$ à $C_4$, trifluorométhyl, en présence d'acide formique en solution dans un solvant tel que l'éthanol.

3. Procédé de préparation des produits selon la revendication 1 tels que $NR_1R_2$ soit égal à $NH_2$ caractérisé en ce que l'on chauffe un mélange constitué par un solvant, de préférence l'éthanol, de l'ammoniac, et une indanedione-1,3 de formule générale

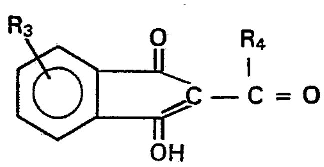

dans laquelle

$R_3$ représente un atome d'hydrogène ou un ou plusieurs substituants, identiques ou différents, en position quelconque sur le cycle aromatique, choisis dans le groupe constitué par chloro, bromo, fluoro, nitro, $SO_2NHCOCH_3$,

$R_4$ représente un groupement alkyl inférieur de $C_1$ à $C_4$ linéaire ou ramifié choisi dans le groupe formé par méthyl, éthyl, propyl, isopropyl, butyl ou bien un substituant phényl.

4. Nouveau médicament caractérisé en ce que le principe actif est constitué par au moins un produit selon la revendictions 1.

5. Nouveau médicament selon la revendication 4 utile pour le traitement des oedèmes et de l'hypertension artérielle.

6. Nouveau médicament selon la revendication 4 utile pour le traitement des états spasmodiques.

7. Nouveau médicament selon la revendication 4 utile en tant que médicament psychotrope.

8. Nouveau médicament selon la revendication 4 utile dans le traitement des états asthmatiques.

9. Nouveau médicament selon la revendication 4 utile dans le traitement de l'angor.

10. Composition pharmaceutique ou vétérinaire caractérisé en ce qu'elle contient à titre de principe actif au moins un produit selon la revendication 1 en association avec un véhicule pharmaceutique ou un excipient approprié.

**Patentansprüche**

1. Neue chemische Verbindungen der allgemeinen Formel

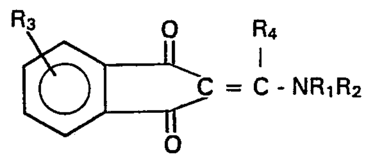

in der

$R_1$ ein Wasserstoffatom oder eine niedrige Alkylgruppe wie Methyl,

$R_2$ ein Wasserstoffatom oder eine geradkettige oder verzweigte, niedrige $C_1$-$C_4$-Alkylgruppe aus der von Methyl, Ethyl, Propyl, Isopropyl, Butyl, Isobutyl und tert.-Butyl gebildeten Gruppe oder eine Alkoxyethyl-, vorzugsweise Methoxyethylgruppe oder eine Cycloalkylgruppe, ausgewählt aus der von Cyclopropyl, Cyclobutyl, Cyclopentyl, Cyclohexyl und Cycloheptyl gebildeten Gruppe oder eine Cycloalkylmethyl-, vorzugsweise Cyclopropylmethylgruppe, oder eine unsubstituierte oder eine am aromatischen Ring mit einem Substituenten oder mehreren aus der von Chlor, Fluor, niedrigem $C_1$-$C_4$-Alkyl, niedrigem $C_1$-$C_4$-Alkoxy und Trifluormethyl gebildeten Gruppe substituierte Benzylgruppe,

$R_3$ ein Wasserstoffatom oder einen in beliebigen Positionen des aromatischen Ringes angeordneten Substituenten aus der von Chlor, Brom, Fluor, Nitro und $SO_2NHCOCH_3$ gebildeten Gruppe oder mehrere dieser Substituenten, und

$R_4$ eine lineare oder verzweigte, niedrige $C_1$-$C_4$-Alkylgruppe aus der von Methyl. Ethyl, Propyl, Isopropyl und Butyl gebildeten Gruppe oder einen Phenylsubstituenten

bedeuten.

2. Verfahren zur Herstellung der Verbindungen nach Anspruch 1, soweit $NR_1R_2$ von $NH_2$ verschieden ist, dadurch gekennzeichnet, dass man ein Indandion-1,3 der allgemeinen Formel

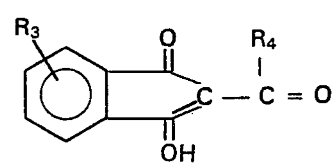

in der

$R_3$ ein Wasserstoffatom oder einen in beliebigen Positionen des aromatischen Ringes befindlichen Substituenten aus der von Chlor, Brom, Fluor, Nitro und $SO_2NHCOCH_3$ gebildeten Gruppe oder mehrere gleiche oder verschiedene dieser Substituenten,

$R_4$ eine lineare oder verzweigte, niedrige $C_1$-$C_4$-Alkylgruppe aus der von Methyl, Ethyl, Propyl, Isopropyl und Butyl gebildeten Gruppe oder einen Phenylsubstituenten bedeuten,

mit einem Amin der allgemeinen Formel $NHR_1R_2$, in der $R_1$ ein Wasserstoffatom oder eine niedrige Alkylgruppe, wie Methyl, $R_2$ eine geradkettige oder verzweigte, niedrige $C_1$-$C_4$-Alkylgruppe, ausgewählt aus der von Methyl, Ethyl, Propyl, Isopropyl, Butyl, Isobutyl,m und tert.-Butyl gebildeten Gruppe oder eine Alkoxyethyl-, vorzugsweise Methoxyethylgruppe oder eine Cycloalkylgruppe, ausgewählt aus der von Cyclopropyl, Cyclobutyl, Cyclohexyl und Cycloheptyl gebildeten Gruppe oder eine Cycloalkylmethyl-, vorzugsweise Cyclopropylmethylgruppe oder eine unsubstituierte oder eine am aromatischen Ring mit einem Substituenten oder mehreren aus der von Chlor, Fluor, niedrigem $C_1$-$C_4$-Alkyl, niedrigem $C_1$-$C_4$-Alkoxy und Trifluormethyl gebildeten Gruppe substituierte Benzylgruppe,
bedeuten,
in Gegenwart von Ameisensäure in Lösung in einem Lösemittel wie Ethanol umsetzt.

3. Verfahren zur Herstellung der Verbindungen nach Anspruch 1, soweit $NR_1R_2 = NH_2$ ist, dadurch gekennzeichnet, dass man ein Gemisch erwärmt, das aus einem Lösemittel, vorzugsweise Ethanol, Ammoniak und einem Indandion-1,3 der allgemeinen Formel

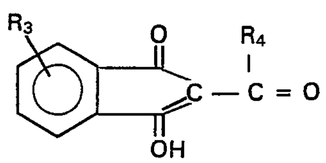

in der
$R_3$ ein Wasserstoffatom oder einen in beliebigen Positionen des aromatischen Ringes angeordneten Substituenten aus der von Chlor, Brom, Fluor, Nitro und $SO_2NHCOCH_3$ gebildeten Gruppe oder mehrere dieser Substituenten, die gleich oder verschieden sein können, und
$R_4$ eine lineare oder verzweigte, niedrige $C_1$-$C_4$-Alkylgruppe aus der von Methyl, Ethyl, Propyl, Isopropyl und Butyl gebildeten Gruppe oder einen Phenylsubstituenten bedeuten.

4. Neues Medikament, dadurch gekennzeichnet, dass es als Wirkstoff mindestens eine Verbindung nach Anspruch 1 enthält.

5. Neues Medikament nach Anspruch 4 zur Verwendung bei der Behandlung von Ödemen und der arteriellen Hypertension.

6. Neues Medikament nach Anspruch 4 zur Verwendung in der Behandlung von spasmischen Zuständen.

7. Neues Medikament nach Anspruch 4 zur Verwendung als psychotropes Medikament.

8. Neues Medikament nach Anspruch 4 zur Verwendung bei der Behandlung von asthmatischen Zuständen.

9. Neues Medikament nach Anspruch 4 zur Verwendung bei der Behandlung von Angstzuständen.

10. Pharmazeutische oder veterinärmedizinische Zusammensetzung, dadurch gekennzeichnet, dass sie als Wirkstoff mindestens eine Verbindung nach Anspruch 1 zusammen mit einem pharmazeutischen Träger oder einem geeigneten Exzipienten enthält.

**Claims**

1. New chemicals with as general formula

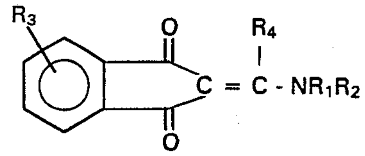

wherein
$R_1$ represents a hydrogen atom or a lower alkyl group such as methyl,
$R_2$ represents a hydrogen atom or a lower alkyl group from $C_1$ to $C_4$, linear or branched, selected from the group formed by methyl, ethyl, propyl, isopropyl, butyl, isobutyl, tertiobutyl, or else an alkoxyethyl group, preferably methoxyethyl, or else a cycloakyl selected from the group formed by cyclopropyl, cyclobutyl, cyclopentyl, cyclohexyl and cycloheptyl, or else a cycloalkylmethyl group, preferably cyclopropylmethyl, or else a benzyl group which is substituted or not on the aromatic cycle with one or a plurality of substituents selected from chloro, bromo, fluoro, lower alkyl from $C_1$ to $C_4$, lower alkoxy from $C_1$ to $C_4$, trifluoromethyl,
$R_3$ represents a hydrogen atom or one or a plurality of substituents in any position on the aromatic cycle, selected from the group comprised of chloro, bromo, fluoro, nitro, $SO_2NHCOCH_3$,
$R_4$ represents a lower alkyl group from $C_1$ to $C_4$, linear or branched, selected from the group formed by methyl, ethyl, propyl, isopropyl, butyl, or else a phenyl substituent.

2. Method for preparing products as defined in claim 1, with $NR_1R_2$ being different from $NH_2$, which comprises reacting an indanedione-1,3 with as general formula

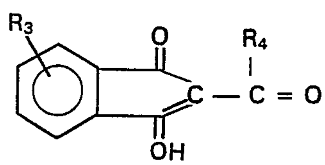

wherein
$R_3$ represents a hydrogen atom or one or a plurality of substituents, identical or different, in any position on the aromatic cycle, selected from the group comprised of chloro, bromo, fluoro, nitro, $SO_2NHCOCH_3$,
$R_4$ represents a lower alkyl group from $C_1$ to $C_4$, linear or branched, selected from the group comprised of methyl, ethyl, propyl, isopropyl, butyl or else a phenyl substituent,

with the amine having as general formula $HNR_1R_2$, wherein $R_1$ represents a hdrogen atom or a lower alkyl group such as methyl, $R_2$ represents a lower alkyl group from $C_1$ to $C_4$, linear or branched, selected from the group formed by methyl, ethyl, propyl, isopropyl, butyl, isobutyl, tertiobutyl, or else an alkoxyethyl group, preferably methoxyethyl, or else a cycloalkyl group selected from the formed by cyclopropyl, cyclobutyl, cyclopentyl, cyclohexyl and cycloheptyl, or else a cycloalkylmethyl group, preferably cyclopropylmethyl, or else a benzyl group, preferably or not on the aromatic cycle with one or a plurality of substituents selected from chloro, bromo, fluoro, lower alkyl from $C_1$ to $C_4$, lower alkoxy from $C_1$ to $C_4$, trifluoromethyl,
in the presence of formic acid in solution in a solvent such as ethanol.

3. Method for preparing prodcuts as defined in claim 1, with $NR_1R_2$ being equal to $NH_2$, which comprises heating a mixture comprised of a solvent, preferably ethanol, ammoniac, and an indanedione-1,3 with as general formula

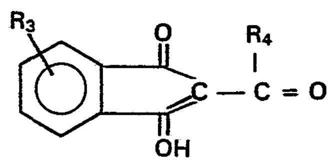

wherein

$R_3$ represents a hydrogen atom or one or a plurality of substituents, identical or different, in any position on the aromatic cycle, selected from the group comprised of chloro, bromo, fluoro, nitro, $SO_2NHCOCH_3$,

$R_4$ represents a lower alkyl group from $C_1$ to $C_4$, linear or branched, selected from the group formed by methyl, ethyl, propyl, isopropyl, butyl, or else a phenyl substituent.

4. New medicament, in which the active constituent is comprised of at least one product as defined in claim 1.

5. New medicament as defined in claim 4, useful for treating oedemae and high blood pressure.

6. New medicament as defined in claim 4, useful for treating spastic conditions.

7. New medicament as defined in claim 4, useful as psychotropic medicament.

8. New medicament as defined in claim 4, useful for treating asthmatic conditions.

9. New medicament as defined in claim 4, useful for treating angor.

10. Pharmaceutical or veterinary composition, which contains as active constituent at least one product as defined in claim 1, associated with a suitable pharmaceutical medium or vehicle.